# EUROPEAN PATENT SPECIFICATION

(11) **EP 3 659 564 B1**
(45) Date of publication and mention of the grant of the patent: **08.10.2025**
(21) Application number: 20153535.8
(22) Date of filing: 08.10.2015
(51) Int. Cl.: A61F 13/551, A61F 13/56, A61F 13/496, A61F 13/49, A61F 13/53

(54) **ADULT DISPOSABLE ABSORBENT ARTICLES AND ARRAYS OF SAID ARTICLES COMPRISING IMPROVED DESIGNS**
SAUGFÄHIGE EINWEGARTIKEL FÜR ERWACHSENE UND ANORDNUNGEN DER BESAGTEN ARTIKEL MIT VERBESSERTEM DESIGN
ARTICLES ABSORBANTS JETABLES POUR ADULTES ET ENSEMBLES DESDITS ARTICLES PRÉSENTANT DES CONCEPTIONS AMÉLIORÉES

(30) Priority: 09.10.2014 US 201462061851 P
(43) Date of publication of application: 03.06.2020
(62) Divisional of application: 15784244.4
(73) Proprietor: The Procter & Gamble Company, Cincinnati, OH 45202 (US)
(72) Inventor: HAMILTON, Raymond Scott, Cincinnati, OH Ohio 45202 (US); MAGEE, Luke Robinson, Cincinnati, OH Ohio 45202 (US); SEITZ, Bret Darren, Cincinnati, OH Ohio 45202 (US); LAVON, Gary Dean, Cincinnati, OH Ohio 45202 (US)
(74) Representative: P&G Patent Germany

(56) References cited:
- US-A1- 2008 110 782
- US-A1- 2013 211 355
- US-A1- 2013 211 357

## Description

### FIELD

The present disclosure is directed to disposable absorbent articles and arrays of disposable absorbent articles which are designed to fit different adult body sizes, shapes and types, and as such are sized to fit a broad range of adult consumers.

### BACKGROUND

Adult incontinence ("AI") articles are designed to absorb and contain liquid and other discharges from the human body to prevent the body and clothing from becoming soiled. Some wearers prefer a pant style that provides the maximum coverage, fully covering the buttocks as well as rising up past and covering the belly button. Other wearers, however, especially younger wearers, prefer lower cut designs as these articles are typically more underwear like and less noticeable under clothing. The challenge, however, is to provide the desired level of leak protection, while providing an article with a smaller silhouette. Another challenge is to provide a line-up of AI articles that meets the needs and stylistic desires of diverse wearers, which can range over several hundred pounds. This challenge is difficult as manufacturers of such products need to keep the number of size offerings to a minimum in order to keep the articles affordable. One of the keys for meeting these challenges is core length and placement relative to product pitch along with other key article parameters, including belt width. The currently marketed AI pant-style articles, including the arrays of AI pant offerings, fail to address proper core placement to protect the wearers to a desired level. Thus, it is an object of the present disclosure to describe absorbent articles and arrays of absorbent articles designed to meet these needs.

US 20130211357 discloses an array of taped and pant absorbent articles comprising substantially identical chassis.

US 20130211355 discloses an array of taped and pant absorbent articles comprising substantially identical chassis.

### BRIEF DESCRIPTION OF THE DRAWINGS

FIG. 1 is a perspective view of an exemplary disposable pull-on garment in a typical in-use configuration;
FIG. 2 is a perspective view of the exemplary disposable pull-on garment of Fig. 1;
FIG. 3 is a plan view of the pull-on garment in its flat uncontracted condition showing the inner surface;
FIG. 4A-4C are schematic cross section views of suitable embodiments taken along line 4-4 in Figure 3;
FIG. 5A-5C are schematic cross section views of suitable cuff embodiments taken along line 5-5 in FIG. 3; and
FIGS. 6-8 are plan views of embodiments of a pull-on garment in its flat uncontracted condition showing their exterior surfaces.

### DETAILED DESCRIPTION

The invention is defined by the claims.

Insofar as the term embodiment is used in the following, or features are presented as being optional, this should be interpreted in such a way that the only protection sought is that of the invention claimed. Reference(s) to "embodiment(s)" throughout the description which are not under the scope of the appended claims merely represent possible exemplary executions and are not part of the present invention.

As used herein, the term "array" means a display of packages comprising disposable articles of different sizes having like article constructions (e.g., same elastomeric materials [compositionally and/or structurally] in the flaps, graphic elements) said packages having the same brand and/or sub-brand, and said packages oriented in proximity to each other in a given area of a retail store. An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Depend," and same sub-brand, for example, "for Women Underwear." A different array may have the brand "Depend" and the sub-brand "Silhouette For Women." The differences between the "for Women Underwear" array and the "Silhouette For Women" arrays include different elastomeric materials in the side flaps, where "for Women Underwear" comprises strands as the elastomeric material and "Silhouette For Women" comprises a film elastomeric material. Furthermore, the packaging is distinctly different in that "for Women Underwear" is packaged in a predominately green, film bag and "Silhouette For Women" is packaged in a predominately maroon box.

Further regarding "arrays," as another example of two separate "arrays" having the same brand, "Certainty," one line-up has the sub-brand "Women's Underwear." A different array may have the same brand "Certainty" and the sub-brand "Smooth Shape Briefs for Women." The differences between the "Women's Underwear" array and the "Smooth Shape Briefs for Women" arrays include different elastomeric materials in the side flaps, where "Women's Underwear" comprises strands as the elastomeric material and "Smooth Shape Briefs for Women" comprises a film elastomeric material. Furthermore, the packaging is distinctly different in that "Women's Underwear" is packaged in a predominately blue, film bag and "Smooth Shape Briefs for Women" is packaged in a predominately maroon box.

Arrays also often have the same trademarks, including trademarks of the brand, sub-brand, and/or features and/or benefits across the line-up.

As used herein, the term "on-line array" means an "array" distributed by a common on-line source.

As used herein, the term "pull-on garment" refers to articles of wear which have a defined waist opening and a pair of leg openings and which are pulled onto the body of the wearer by inserting the legs into the leg openings and pulling the article up over the waist. The term "disposable" is used herein to describe garments which are not intended to be laundered or otherwise restored or reused as a garment (i.e., they are intended to be discarded after a single use and to be recycled, composted or otherwise disposed of in an environmentally compatible manner). The pull-on garment may be "absorbent" such that it absorbs and contains the various exudates discharged from the body.

As used herein, the term "absorbent article" refers to pull-on garments worn by incontinent individuals, including adults, to absorb and contain urine, feces and/or menses. It should be understood, however, that the term absorbent article is also applicable to other garments such as incontinent briefs, feminine hygiene garments or panties, and the like.

As used herein, the term "belt" refers to waistband, ears, side-panels, back panels, etc. For instance, while the present disclosure illustrates articles comprising belt-style articles, the articles may alternatively comprise flaps as disclosed in U.S.S.N. 61/990,327, titled LENGTH-TO-HIP SILHOUETTES OF ADULT DISPOSABLE ABSORBENT ARTICLES AND ARRAYS, to Seitz, et al.

As used herein, the terms "elastic," "elastomer," and "elastomeric" refer to a material which generally is able to extend to a strain of at least 50% without breaking or rupturing, and is able to recover substantially to its original dimensions, accounting for set, after the deforming force has been removed.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

Please note that throughout the specification, structural elements of the present disclosure may be referred to generally, like side edges 47, which is meant to encompass side edge 47a and side edge 47b.

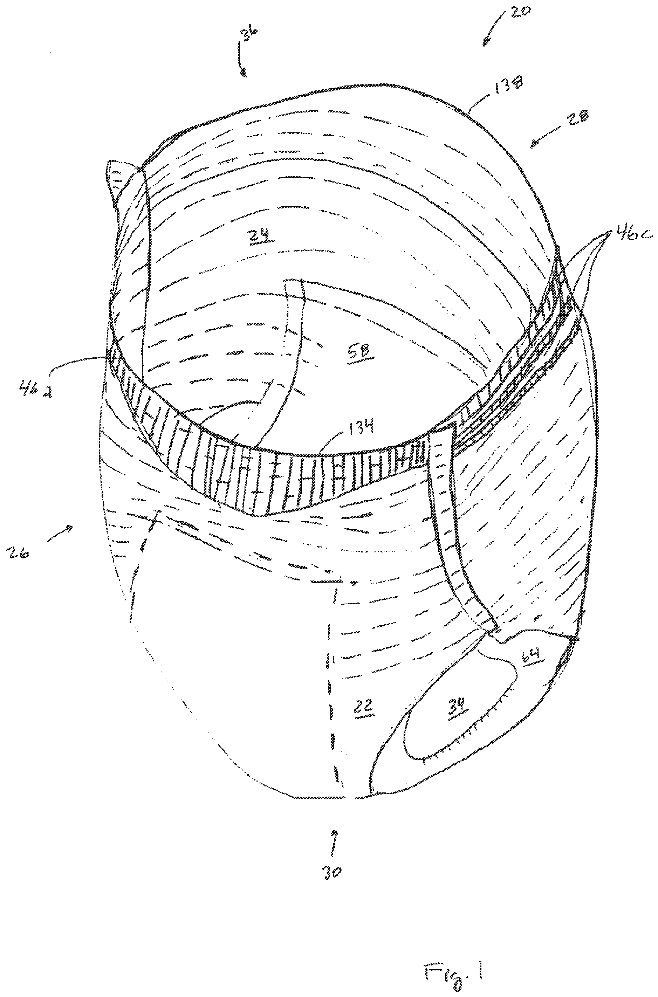

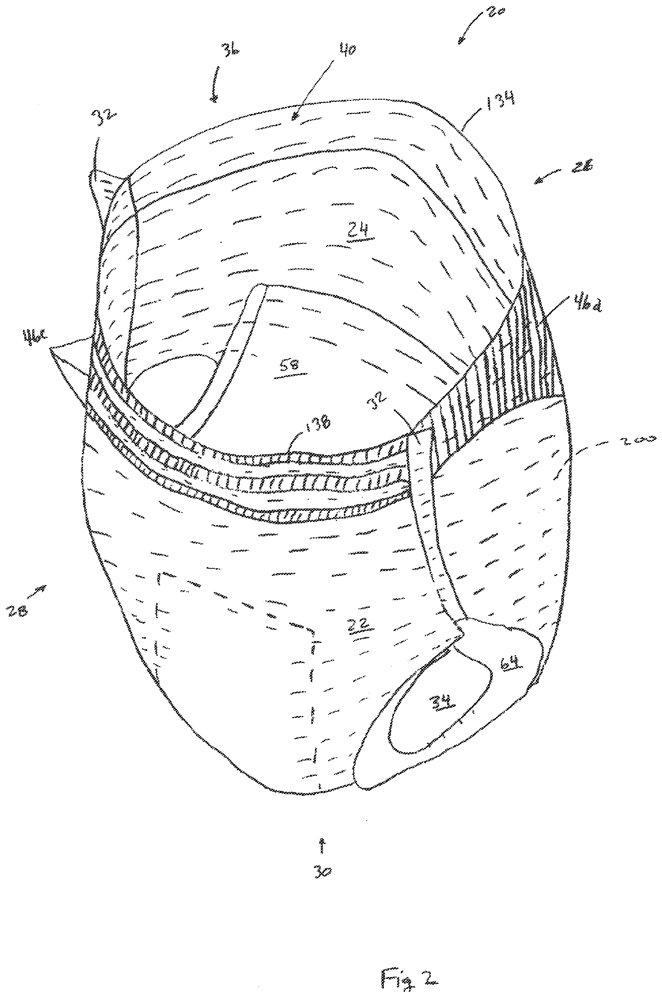

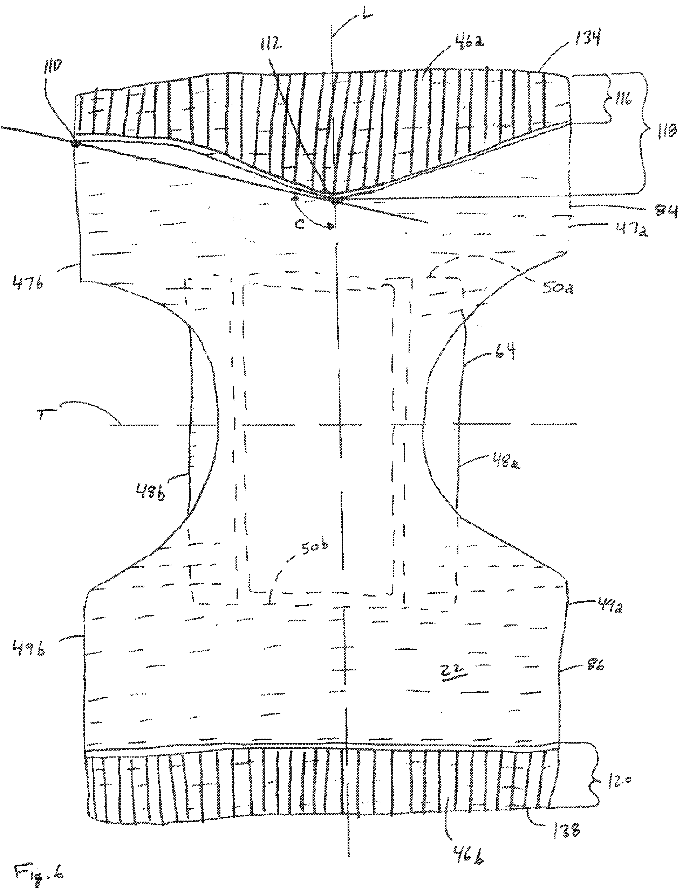

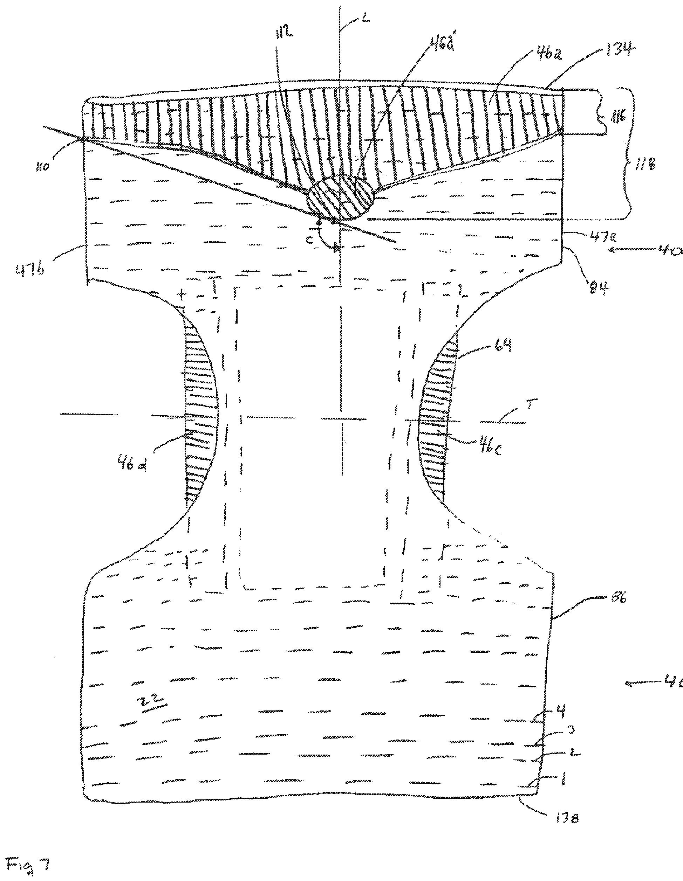

Figures 1 and 2 are perspective views of the absorbent article 20. As shown in Figs. 6 and 7, the absorbent article 20 has a longitudinal centerline L and a transverse centerline T. The absorbent article 20 has an outer (or "exterior") surface 22, an inner surface 24 opposed to the outer surface 22, a front region 26 (or "front waist region"), a back region 28 ("or back waist region"), a crotch region 30, and seams 32 which join the front region 26 and the back region 28 to form two leg openings 34 and a waist opening 36.

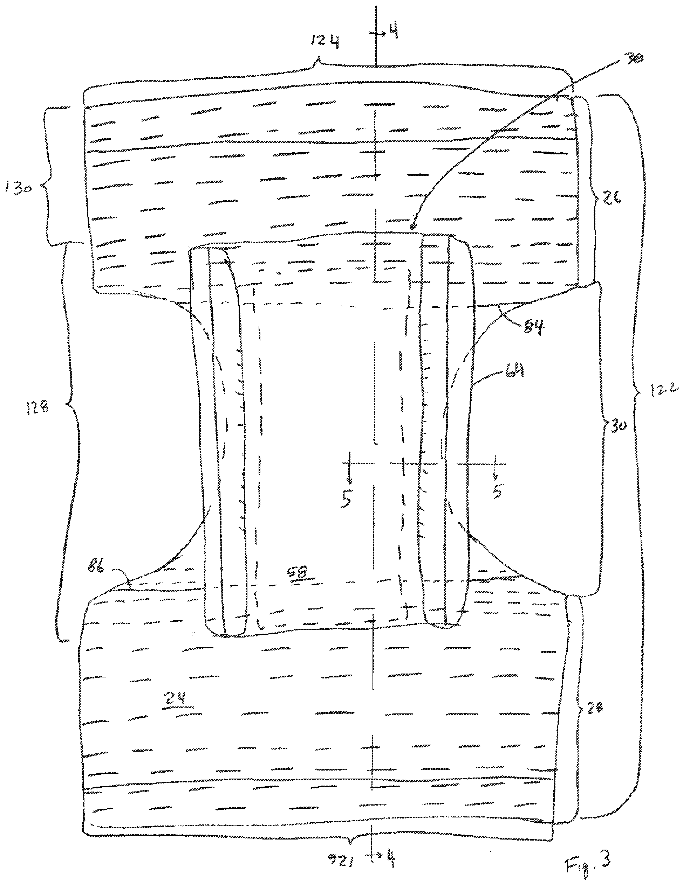

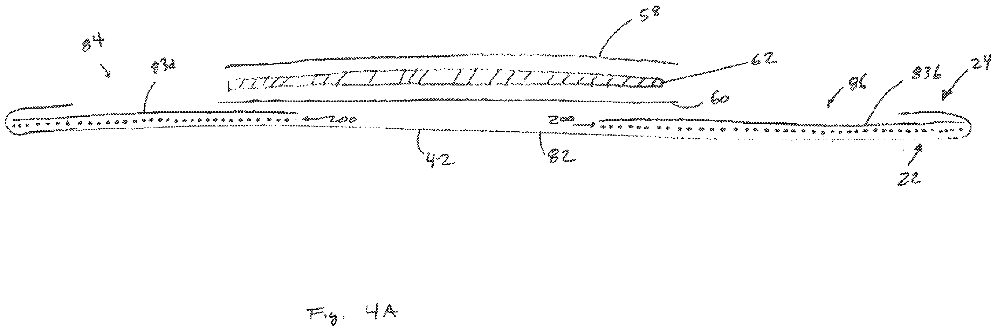

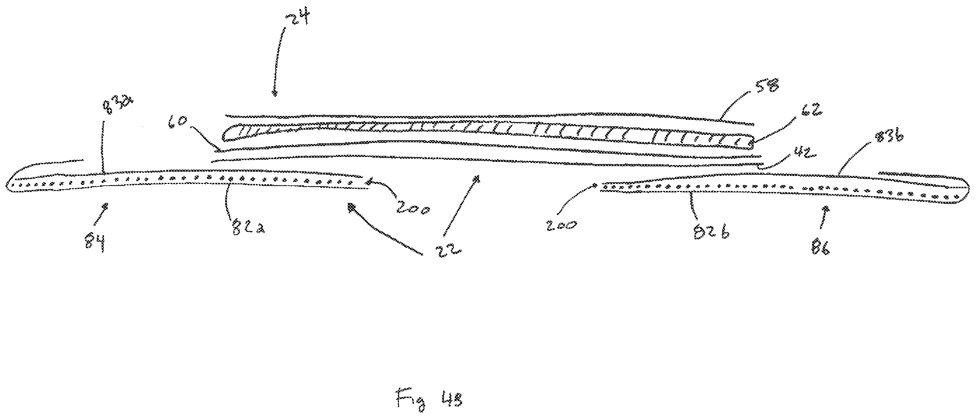

In the embodiment shown in Figures 1 and 3, the absorbent article 20 comprises an absorbent main body 38 (hereinafter may be referred to as "main body" or "center chassis") to cover the crotch area of the wearer and a belt 40 extending transversely about the waist opening 36. As shown in Figs. 4A-C, the absorbent article 20 may also comprise an outer cover layer 42 to cover the main body 38. The belt 40 defines the waist opening 36. The belt 40, the main body 38 and/or the outer cover layer 42, and/or the cuffs 64 may jointly define the leg opening 34.

In the embodiment shown in Figure 2 the absorbent article 20 comprises an absorbent main body 38 to cover the crotch area of the wearer and a belt 40 extending transversely about the waist opening 36. The absorbent article 20 may also comprise an outer cover layer 42 to cover the main body 38. The belt 40 defines the waist opening 36. In this embodiment, the belt 40, the main body 38 and/or the outer cover layer 42 jointly define the leg opening 34. One or more of the belt layers may extend from a first waist edge 134 in a first waist region 26 through the crotch region 30 to a longitudinally opposing second waist edge 138 in a second waist region 28 and may form a portion or the whole of the outer surface of the absorbent article 20.

The absorbent main body 38 absorbs and contains body exudates. In the embodiment shown in Figs. 6 and 7, the main body 38 has a generally rectangular shape having a longitudinal centerline L, a transverse centerline T, left and right longitudinally extending side edges 48a and b (hereinafter may be referred to as "longitudinal side edge(s)") and front and back transversely extending end edges 50a and 50 (hereinafter may be referred to as "transverse end edge(s)").

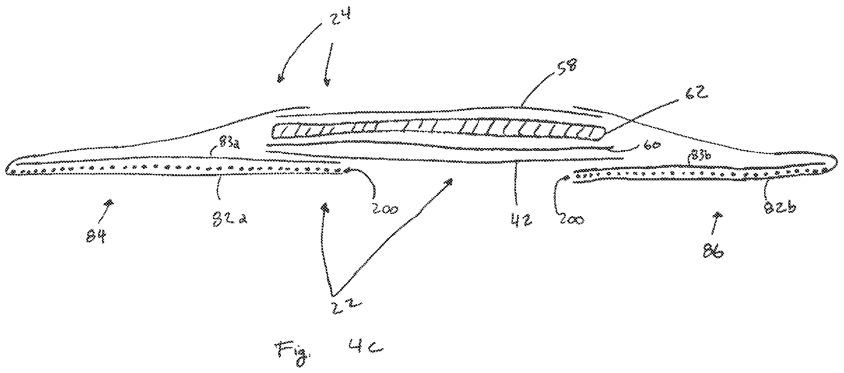

In the embodiments shown in Figs. 4A-C, the absorbent articles 20 may comprises front and rear (or "back") extensible belts 84, 86 disposed in the front and rear (or "back") waist regions 26, 28 respectively (and in some embodiments may extend into the crotch region 30) and intended to encircle at least a portion of the waist of the wearer, the front and rear belts 84, 86 being connected by the main body 38. The front and rear belts 84 and 86 may be formed from a first outer belt layer 82 (that may also serve as the outer cover layer 42 of the main body 38) and may extend from a first waist edge 134 in a first waist region 26 through the crotch region 30 to a longitudinally opposed second waist edge 138 in a second waist region 28 and forming a portion or the entire outer surface 22 of the absorbent article 20. Alternatively, the outer belt layer 82 may have a front portion 82a longitudinally separated from a back portion 82b. The outer belt layer may wrap the front waist edge 134 and the rear waist edge 138 and overlap second inner belt layers 83a and 83b or may overlap a portion of the main body 38 (including the topsheet 58, as shown in Fig. 4C). The inner belt layer 83 may also be continuous from the front waist edge 134 to the rear waist edge 138.

The second belt layer 83a and b (e.g., an "inner belt web(s)") may form a portion of the inner surface 24 of the absorbent article 20. The second belt layer 83 may be formed of two longitudinally spaced webs of material. The first and second belt portions may also comprise an elastomeric material 200 (e.g., "elastic elements" or "elastics") disposed between the first and second belt layers. The elastomeric material may comprise elastic strands, elastomeric films, elastomeric ribbons, elastomeric nonwovens, elastomeric filaments, elastomeric adhesives, elastomeric foams, scrims, apertured films (as described in U.S. Patent Nos. 6,410,129; 7,087,287; and U.S. Pub. No. 2007-0287348), or combinations thereof. A portion of the elastomeric material 200 may be directly combined with the outer cover layer. The main body 38 ("central or center chassis") of the absorbent article may comprise a backsheet 60, a topsheet 58, and an absorbent core 62 disposed between the topsheet 58 and the backsheet 60. The main body 38, as well as the first belt layer 82, may form a portion of the outer surface 22 as shown in Fig. 4C. The main body, as well as the first and second belt layers 82 and 83, may form a portion of the inner surface 24. In addition, the main body 38 may comprise elasticized cuffs 64 disposed at or adjacent to (or may form) the side edges 48 of the main body 38.

The first and second belt layers 82, 83 may be formed of substantially the same material or may comprise different materials. Likewise, the front and rear belts 84, 86 may be formed of substantially the same material or may comprise different materials. The first and second belt layers 82 and 83 may be formed from nonwovens, films, apertured films (as described in U.S. Patent Nos. 6,410,129; 7,087,287; and U.S. Pub. No. 2007-0287348), foams, woven materials or combinations thereof.

Additional lateral extensibility in the main body 38 and/or the front and rear belts 84,86 (making up the belt 40) may be provided in a variety of ways. For example, a material or materials from which the main body 38 and/or the belt 40 is made may be pleated by any of many known methods. Alternatively, all or a portion of the main body 38 may be made of a formed web material or a formed laminate of web materials like those described in U.S. Patent No. 5,518,801 issued on 21 May 1996 in the name of Chappell *et al.* This formed web material includes distinct laterally extending regions in which the original material has been altered by embossing or another method of deformation to create a pattern of generally longitudinally oriented alternating ridges and valleys and also includes laterally extending unaltered regions between the laterally extending altered regions. The formed web material can be extended in a direction perpendicular to the ridges up to the point where the ridges and valleys flatten with substantially less force than is required to extend beyond that point. In addition to lateral extensibility, the creation of a formed laminate web as described above provides a main body 38, backsheet 60 and or the outer cover nonwovens 42, 82 with improved texture and cloth-like appearance and feel. The deformation creates a cloth-like pattern in the film and/or the nonwovens and increases the loft of the nonwoven in multi-layer film and nonwoven laminate backsheets.

Alternatively, a portion of the absorbent article can be ring-rolled and thus rendered highly extensible as described in U.S. Pat. No. 5,366,782 (issued Nov. 22, 1994 to Curro, et al). Specifically, a ring-rolling apparatus includes opposing rolls having intermeshing teeth that incrementally stretch and thereby plastically deform the material forming the absorbent article (or a portion thereof) thereby rendering the article extensible in the ring-rolled regions. In one embodiment, portions of the absorbent article 20 can be ring-rolled in a portion of at least one of the front or rear waist regions, for example the portion of the main body 38 underlying and/or immediately adjacent one or both of the front and rear belts 84, 86, while other regions may comprise a structured elastic-like formed web material. The article may be ring-rolled across the entire width in one or both of the waist regions or alternatively may be ring-rolled over only a portion of the main body 38 width or over only a portion of one or both of the belts 84, 86.

### TOPSHEET

In one embodiment, the absorbent article 20 may comprise a topsheet 58. The topsheet 58 may be compliant, soft feeling, and non-irritating to the wearer's skin and may be elastically stretchable in one or more directions. Further, the topsheet 58 may be liquid pervious, permitting liquids (e.g., menses, urine, and/or runny feces) to penetrate through its thickness. Various topsheets may also comprise a hydrophilic material, for example, which is configured to draw bodily fluids into an absorbent core 62 when these fluids are expelled from the body. A suitable topsheet 58 may be manufactured from a wide range of materials, such as woven and nonwoven materials, apertured or hydroformed thermoplastic films, apertured nonwovens, porous foams, reticulated foams, reticulated thermoplastic films, and/or thermoplastic scrims, for example. Suitable apertured films may comprise those described in U.S. Pat. Nos. 3,929,135, 4,324,246, 4,342,314, 4,463,045, 5,006,394, 5,628,097, 5,916,661, 6,545,197, and 6,107,539.

Apertured film or nonwoven topsheets typically may be pervious to bodily exudates, yet non-absorbent, and have a reduced tendency to allow fluids to pass back through and rewet the wearer's skin. Suitable woven and nonwoven materials may comprise natural fibers, such as, for example, wood or cotton fibers, synthetic fibers, such as, for example, polyester, polypropylene, or polyethylene fibers, or combinations thereof. If the topsheet 58 comprises fibers, the fibers may be spunbond, carded, wet-laid, meltblown, hydroentangled, or otherwise processed, for example, as is generally known in the art.

The topsheet 58 may comprise a skin care lotion. Examples of suitable lotions include, but are not limited to, those described in U.S. Pat. Nos. 5,607,760; 5,609,587; 5,635,191; 5,643,588; and 5,968,025, and as described in U.S. Application No. 61/391,353, and as described in U.S. Pub. No. 2014-0257216. Beyond these compositions, the absorbent article may comprise soluble cyclodextrin derivatives such as those described in U.S. Pub. No. 2014/0274870.

Additionally, the topsheet of the present disclosure may be a tufted laminate web as disclosed in U.S. Pat. No. 7,410,683, and/or may be an apertured web as disclosed in PCT/CN2014/083769 having an international filing date of August 6, 2014.

### ABSORBENT CORE

In various embodiments, the absorbent article 20 may comprise an absorbent core (also referred to as an "absorbent member" or "absorbent assembly" or "absorbent structure" or "absorbent composite") 62 that is disposed between the topsheet 58 and the backsheet 60. In one embodiment, more than one absorbent core 62 or more than one absorbent core layer may be provided in an absorbent article 20, for example. Suitable absorbent cores that may be used are disclosed in U.S. Pat. Nos. 4,610,678; 4,673,402; 4,888,231; and 4,834,735.

In one embodiment, the absorbent core 62 may comprise cellulosic airfelt material. For instance, such absorbent cores may comprise less than about 40%, 30%, 20%, 10%, 5%, or even 1% of the cellulosic airfelt material as determined by weight. Additionally, such an absorbent core may be primarily comprised of an absorbent gelling material in amounts of at least about 60%, 70%, 80%, 85%, 90%, 95%, or even about 100% as determined by weight. Furthermore, a portion of the absorbent core may comprise a microfiber glue (if applicable). Such absorbent cores, microfiber glues, and absorbent gelling materials are described in U.S. Pat. Nos. 5,599,335; 5,562,646; 5,669,894; 6,790,798; and 7,521,587 and in U.S. Pat. Publ. No. 2004/0158212.

In one embodiment, the core, including multiple layers making up a core system, may be printed and embossed as described in U.S. Pat. No. 8,536,401.

In one embodiment, the core may be separable from the chassis as disclosed in U.S. Pat. Nos. 6,989,006; 7,381,202; 7,175,613; 7,824,386; 7,766,887; and 6,989,005.

In one embodiment, the absorbent article 20 of the present disclosure, and particularly, a portion where the absorbent member is disposed, may have a body fluid absorption rate greater than 3 g/sec according to U.S. Pat. No. 6,649,810. According to U.S. Pat. No. 6,649,810, the expression "the portion (of the absorbent article) where the absorbent member is disposed" is intended to mean the portion occupied by the absorbent member when the absorbent article is flatly unfolded and seen in its plan view.

In one embodiment, the absorbent structure may have an intake factor greater than 3 according to U.S. Pat. No. 7,073,373, wherein the intake factor is defined as the absorbent core permeability divided by the normalized retention capacity (which is defined by the Retention Capacity Test - also according to U.S. Pat. No. 7,073, 373).

In one embodiment, the absorbent composite has a body fluid absorption greater than 75 g/100 cm², according to U.S. Pat. No. 6,649,810.

In one embodiment, a target location of the absorbent article may have a wicking value greater than 36%, according to U.S. Pat. No. 6,383,960.

In one embodiment, the absorbent article may have a bending stiffness between 0.05-1.0 gf, according to U.S. Pat. No. 5,810,796.

In one embodiment, the absorbent article may have a crotch fluid absorption rate greater than 3g/sec according to U.S. Pat. No. 6,649,810. In one embodiment, a freeze-dried composite of the absorbent composite may have an intake rate of at least about 1.9 cubic centimeters (cc) of liquid/second at 80% composite saturation according to U.S. Pat. No. 6,689,934.

In one embodiment, the absorbent core 62 may comprise channels as described in U.S. Pat. No. 8,568,566; U.S. Pub. Nos. 2012-316046, 2014-027066, 2014-163500, 2014-163506, 2014-163511, 2012-316526, 2012-316527, 2012-316528, 2012-316529, 2012-316523, 2014-163501, 2014-163502, 2014-163503; and European Pub. Nos. 2532328, 2532329, 2717823, 2717820, 2717821, 2717822, 2532332, 2740449, and 2740452.

### BACKSHEET

The absorbent article 20 may comprise a backsheet 60. The backsheet 60 may be impervious, or at least partially impervious, to fluids or body exudates (e.g., menses, urine, and/or runny feces) and may be manufactured from a thin plastic film, although other flexible liquid impervious materials may also be used. The backsheet 60 may prevent the bodily exudates or fluids absorbed and contained in an absorbent core 62 of the absorbent article 20 from wetting articles of clothing that contact the absorbent article 20. The backsheet 60 may comprise a woven or nonwoven material, polymeric films such as thermoplastic films of polyethylene or polypropylene, and/or a multi-layer or composite materials comprising a film and a nonwoven material (e.g., having an inner film layer 60 and an outer nonwoven layer 42). A suitable backsheet may comprise a polyethylene film having a thickness of from about 0.012 mm (0.5 mils) to about 0.051 mm (2.0 mils). Examples of polyethylene films are manufactured by Clopay Corporation of Cincinnati, Ohio, under the designation BR-120 and BR-121, and by Tredegar Film Products of Terre Haute, Ind., under the designation XP-39385.

One suitable material for the backsheet 60 can be a liquid impervious thermoplastic film having a thickness of from about 0.012 mm (0.50 mil) to about 0.051 mm (2.0 mils), for example including polyethylene or polypropylene. Typically, the backsheet 60 can have a basis weight of from about 5 g/m² to about 35 g/m². The backsheet 60 can be typically positioned adjacent the outer-facing surface of the absorbent core and can be joined thereto. For example, the backsheet 60 may be secured to the absorbent core 62 by a uniform continuous layer of adhesive, a patterned layer of adhesive, or an array of separate lines, spirals, or spots of adhesive. Illustrative, but non-limiting adhesives, include adhesives manufactured by H. B. Fuller Company of St. Paul, Minn., U.S.A., and marketed as HL-1358J. An example of a suitable attachment device including an open pattern network of filaments of adhesive is disclosed in U.S. Pat. No. 4,573,986. Another suitable attachment device including several lines of adhesive filaments swirled into a spiral pattern is illustrated by the apparatus and methods shown in U.S. Pat. Nos. 3,911,173; 4,785,996; and 4,842,666. Alternatively, the attachment device may include heat bonds, pressure bonds, ultrasonic bonds, dynamic mechanical bonds, or any other suitable attachment device or combinations of these attachment devices.

In one embodiment, the backsheet 60 may be embossed and/or matte-finished to provide a more cloth-like appearance. Further, the backsheet 60 may permit vapors to escape from the absorbent core 62 of the absorbent article 20 (such that the backsheet 60 is breathable) while still preventing, or at least inhibiting, fluids or body exudates from passing through the backsheet 60.

### LEG CUFFS

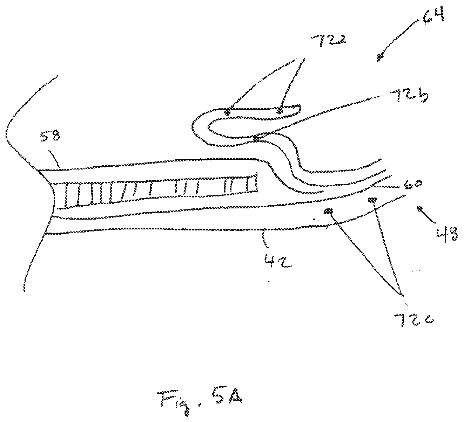

The cuff 64 (inner cuff 64a and outer cuff 64b) provides improved containment of liquids and other body exudates. A suitable embodiment of the cuff 64 shown in Figs. 5A-C comprises a single layer of material which may be folded to form a barrier leg cuff having two layers. The cuff 64 extends from the side of the main body at or adjacent, or forming part of, the longitudinal side edge 48 toward the longitudinal centerline L.

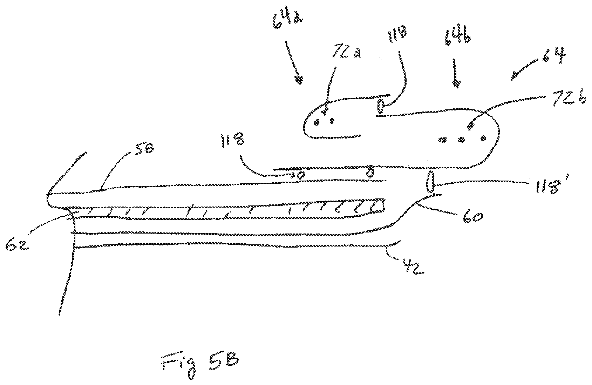

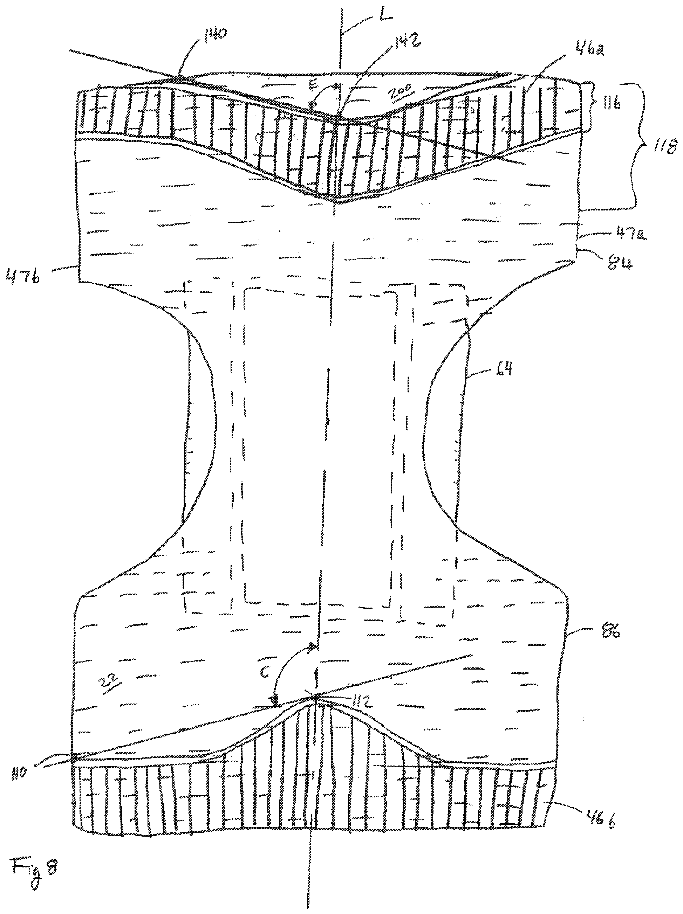

The cuff 64 may have first, second, and third barrier cuff elastic materials 72a, b, and c; each of the elastic materials may be the same or different. A distal portion of the cuff 64 may be adhered to a distal portion of the backsheet film 60, and another portion of the cuff 64 may be adhered to the topsheet via adhesive 118 as illustrated in Figs. 5A-C. Beyond these cuff 64 configurations, other suitable examples of cuffs 64 that may be used herein are disclosed in U.S.S.N. 13/457,521, filed April 27, 2012, including the configurations disclosed by Figures 8a-t. For instance, as illustrated in Fig. 5B, the cuff may be two-piece. And, the cuff 64 may be joined to the backsheet with a no leak bead 118' that runs along the entire longitudinal length of the cuff 64 and/or the backsheet film 60.

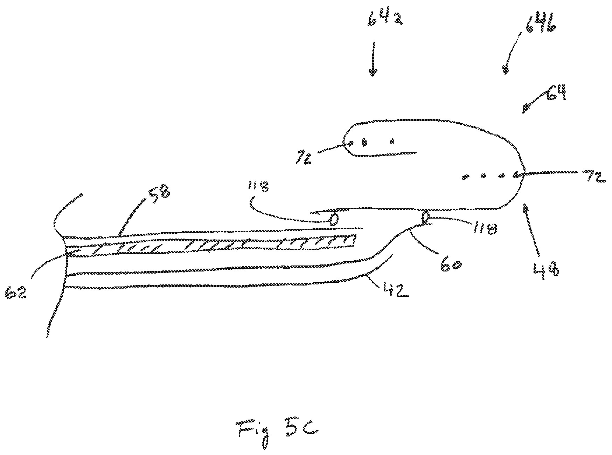

As shown in Fig. 5A, the backsheet film 60, the outercover nonwoven 42, and both layers of the cuff 64 may co-terminate at the side edge 48. Alternatively, as shown in Figs. 5B and C, the distal end of the cuff 64 may extend beyond the other materials to form at least a portion of the side edge 48 in a manner that exposes at least a portion of the cuff when the article 20 is worn, such that a more finished folded leg edge is achieved.

Other suitable cuffs 64 may be configured as those described in U.S. Pat. Nos. 3,860,003, 4,909,803, 4,695,278, 4,795,454, 4,704,115, and 4,909,803, and U.S. Pat. Publ. No. 2009/0312730.

### GRAPHICS

The belts 84 and 86 may comprise graphics 46a, 46a', 46b, and 46c, including graphic objects and graphic patterns as disclosed in U.S. Publication Nos. 2011/0203102 and 2011/0192010, such that graphics 46 may form the appearance of a waistband around the entire, or substantially the entire, belt 40 as illustrated in Figs. 1, 2 and 6. Further, the graphics of the present disclosure may be printed in the same manner as described in U.S. Publication Nos. 2011/0203102 and 2011/0192010, and may be printed on the exterior surface 22 of the article 20, or may be printed on an interior surface of the outer belt layer 82, or may be printed on the garment-facing surface of the inner belt 83, or may be printed on a sheet (not shown) placed between the belt layers 82, 83. Further, the articles 20 disclosed herein may have graphics in accordance with U.S.S.N.s 61/646,953 and 61/646,979, each filed on May 15, 2012.

Graphics 46a and b may be disposed on the front and rear regions 26, 28 and may cooperate to form the appearance of a waistband. As shown in Figs.1, 2, 6 and 7, the graphics may increase in longitudinal distance as they approach the longitudinal centerline L. For instance, as shown in Figs. 6 and 7, a graphic waistband may have a longitudinal distance 116 adjacent to the side seam, but may have a distance 118, which is greater than 116, at or near the longitudinal centerline L. In this way, the graphic 46a may taper as it nears the side seams 32a and b, or the side edges 47a and b of the front belt 84 and side edges 49 a and b of the rear belt 86. This may give the appearance of a better fitting article, and may be especially advantageous when disposed on an article designed to fit under the wearer's bellybutton. Graphic designs that may be desirable for articles 20 of the present disclosure may have a longitudinal distance 116 adjacent the side edge 47 greater than about 15 mm, 25 mm, 50 mm, or about 75 mm, and may have a longitudinal distance 118, that is greater than the distance 116 at the side edge 47, but less than about 150 mm, 100 mm, or about 75 mm, including ranges in any combination of these.

Further, as shown in Fig. 6, the most longitudinally distal point adjacent the side seam 110 to the most longitudinally distal point 112 of a front or back graphic 46a or b may have an angle C that is at least about 110°, 120°, or about 130°, but not more than about 140°, relative to the longitudinal axis L. Alternatively, the back graphic 46b may be generally parallel with the transverse axis T. While Fig. 6 illustrates a graphic 46b that is generally parallel with the transverse axis T, graphic 46b may be replaced with a tapering graphic like the graphic 46a on the front belt 84, such that both graphics 46a and 46b on the front and back belts 84 and 86 are tapering graphics and such that the longitudinal distances 116 and 120 of the side edges 47 and 49 are about the same such that they may line up with each other.

As illustrated in Fig. 7, the back belt 86 may have colored elastic strands adjacent to the back waist edge 138, such that the colored strands (e.g., elastic strands 1-4 of the elastics 200) cooperate with the front belt 84 graphics 46a to form the appearance of a waistband. The number of colored elastic strands may be 1, 2, 3, 4, 5, 6, or 7 in the front belt 84, and/or 1, 2, 3, 4, 5, 6, or 7 colored elastic strands in the back belt 86, for example. The colored strands may be visible through the nonwoven outer cover layer 42, 82 such that they can be seen as part of the outer surface 22 of the absorbent article 20. The colored strands may be color coordinated to match one or more colors of the front belt 84 graphic 46a - the colored strands may be multiple colors to accomplish this. For example, a first colored strand may be blue, a second colored strand may be red, and a third elastic strand may be green. Instead of colored strands, the back belt may comprise bands of graphics 46c, as illustrated in Figs. 1 and 2.

The graphics 46a and b may be disposed into the seam 32, or to the very edge of the side edges 47, 49, or may stop outside of the seam 32.

A portion of the graphic 46a' of the front belt 84 may comprise an element that anchors the front belt 84 graphic 46a, such as a bow, or a knot. For example, the front belt 84 graphic 46a may be in the form of the appearance of lace, while the anchoring element 46a' is the appearance of a bow. This would be an example of a feminine graphic that functions to communicate a low cut panty and that may function to persuade the eye to see more of an extreme low cut feature (i.e., bikini-like underwear look) than there really is.

Further, the leg cuffs 64 may be colored or may comprise a graphic 46c and d that cooperates with the front belt 84 and/or back belt 86 graphics 46a, b, and c. This is especially beneficial in embodiments like illustrated in Figs. 5B, 5C, and 7, where at least a portion of the leg cuffs 64 extends beyond the backsheet 60 or the nonwoven outer cover layer 42.

Referring to Fig. 8, in some embodiments, it may be desirable to have a graphic that not only slopes from its most longitudinally distal point adjacent the side seam (e.g., 110) to the most longitudinally distal point (e.g., 112) such as graphic 46a in Fig. 7, but it may also be desirable to have the front or back belt graphic 46a or b slope from its most proximal point adjacent the side seam 140 to the most longitudinally proximal point of the graphic 142, such that an angle E is at least about 30°, 40°, 50°, or about 60°, but not more than about 90° relative to the longitudinal axis L.

### ODOR CONTROL AGENTS

Absorbent articles 20 of the present disclosure may also comprise odor control agents, including reactive aldehydes, and including the compositions disclosed in PCT/US2014/042892 having an international filing date of June 18, 2014. These agents may be disposed within the core 62, or on the topsheet 58 (including the garment-facing surface of the topsheet), or on the backsheet film 60 (including the body-facing surface of the backsheet).

### REFASTENABLE

The front and back belts 84 and 86 may be permanently or refastenably connected at the seams 32. Regarding refastenable embodiments, articles 20 of the present disclosure may have refastenable elements, configurations, and methods of making as disclosed in U.S.S.N.s 61/787,416, filed on March 15, 2013, as well as U.S.S.N. 61/787,332, filed on March 15, 2013. The refastenable elements may be fastened during the manufacturing process and/or fastened in the package prior to use by the wearer or caregiver (i.e., the article may be sold in "closed form"). The front and back belts 84 and 86 may be in a number of configurations as described and illustrated in Figs. 3A-C and 4A-k of U.S.S.N. 61/666,065, filed on June 29, 2012, titled DISPOSABLE ABSORBENT REFASTENABLE PANTS AND METHODS FOR MANUFACTURING THE SAME. Further, the absorbent articles of this disclosure may be manufactured in accordance with the descriptions and illustrations of U.S.S.N. 61/666,065 (see, for example, Figs. 5-10C of the '065 application).

As illustrated, the belt 40 may be ring-like and elastic. The ring-like elastic belt 40 extends transversely about the waist opening 36 of the absorbent article 20 and acts to dynamically create and distribute forces dynamically generated during wear. Applicants have found that improved fit can be created by controlling the distance between, linear density, and the pre-strain of the elastomeric material in relation to each other and to the openings for the body. This may occur by choosing different materials throughout the belt 40 that exhibit desired properties. The different materials are combined at specific distances, linear densities, and prestrains to create a belt 40 that acts dynamically. Particularly, the articles 20 of this disclosure may have the characteristics of the articles of Examples 1-4 as disclosed in U.S.S.N. 13/764,990, filed February 12, 2013. Articles of the present disclosure may also have the same stress, strain and spacing of its elastics as disclosed in U.S.S.N. 13/764,990 and/or as disclosed in U.S. Serial No. 61/598,012, filed February 13, 2012. Articles 20 of the present disclosure may also have the same elastic sections and force zones disclosed in U.S.S.N. 13/764,990.

The belt 40 of this disclosure may comprise elasticized sections having the elastic profile combinations as disclosed in U.S. Pub. No. 2013/0211363, filed on February 12, 2013, including elastic spacing, dtex, strains, border areas, spacer placement, force zones, force profiles, numbers of elastics, gap distance between the elastic strands, and the articles of the present disclosure may particularly have the elastic profiles as disclosed in Examples 1, 2, and 3 of U.S. Pub. No. 2013/0211363.

Further, the elasticized belts may be in a number of configurations as described and illustrated in Figs. 3A-C and 4A-K of U.S. Pub. No. 2014/0005628, filed on June 28, 2013, titled DISPOSABLE ABSORBENT REFASTENABLE PANTS AND METHODS FOR MANUFACTURING THE SAME. Further, the absorbent articles of this disclosure may be manufactured in accordance with the descriptions and illustrations of U.S. Pub. No. 2014/0005628 (see, for example, Figs. 5-10C of the '628 publication).

It may be desirable to use the hot air seaming processes, as well as the article forming processes disclosed in U.S. Pat. No. 6,248,195 and U.S.S.N.s 12/795021, 13/401907, and 13/402056 for seaming articles as disclosed herein.

The articles 20 of the present disclosure may have Leg Hoop Moduluses, Leg Hoop Forces, and Array Leg Hoop Moduluses as disclosed in U.S.S.N. 61/976,668, filed April 8, 2014, titled Array of Dispable Absorbent Articles For Fitting Broad Range Of Wearers, including the particular moduluses disclosed in Tables 1-4 of the '668 application.

Absorbent articles as disclosed herein may be manufactured by the same company on the same or different manufacturing line(s) and may sold in an array under the same brand (e.g., Pampers, Huggies, Depends, Always) and/or sub-brand name (Cruisers, Swaddlers, and Easy Ups, Baby Dry, Silhouette, etc.).

### IDENTICAL OR SUBSTANTIALLY IDENTICAL CHASSIS

As disclosed in U.S. Pub. No. 2013-0211355, it may be desirable to offer an array of packages for fitting different sized wearers, but comprising identical or substantially identical chassis. For instance, an array may comprise a first package comprising a first size of absorbent articles 20 and a second package may comprise a second size of absorbent articles 20, where the first and second packages comprise identical or substantially identical center chassis 38 as described in U.S. Pub. No. 2013-0211355. More particularly, the first package may comprise a first center chassis 38 and the second package may comprise a second center chassis 38, where each of the first and second center chassis 38 comprise the same dimensions of one or more of: core width at the lateral centerline, core width at one of the front or rear core end, a distance from a left outer cuff distal edge to a right outer cuff distal edge, a distance from a left inner cuff distal edge to a left outer cuff distal edge, a distance from a left inner cuff proximal edge to a right inner cuff proximal edge, a distance from a left inner cuff proximal edge to a left outer cuff distal edge, a free height of the inner cuff, inner cuff hem fold width, inner cuff elastics length, outer cuff elastics length, core length, and backsheet width, as disclosed in U.S. Pub. No. 2013-0211355.

Further, each of the first and second chassis 38 may comprise identical chemical compositions of one or more of a topsheet 58, backsheet film 60, backsheet nonwoven 42, core super absorbent polymers, core pulp, core nonwoven, core tissue, leg cuff film, leg cuff nonwoven, super absorbent polymer adhesive, core nonwoven adhesive, leg cuff elastic adhesive, and backsheet nonwoven/film adhesive.

And, each of the first and second chassis 38 may comprise the same basis weight of one or more of the topsheet, backsheet film, backsheet nonwoven, core super absorbent polymers, core pulp, leg cuff nonwoven, leg cuff film, super absorbent polymer adhesive, leg cuff adhesive, and backsheet nonwoven/film adhesive.

And, each of the first and second chassis 38 may comprise compositionally identical core super absorbent polymers. The first and second chassis 38 may have identical component cross sectional order and disposition in at least one of the front region 26, back region 28, and crotch region 30. The leg cuffs 64 of the first and second chassis 38 may be composed of the compositionally identical materials.

And, the core adhesives of the first and second chassis 38 may be the same adhesive(s). The first and second chassis 38 may comprise core super absorbent polymers that are in the same chemical class and subclass.

And, each of the first and second chassis 38 may comprise first and second wetness indicators, respectively, and wherein the first and second wetness indicators are compositionally identical. The wetness indicators may be one color pre insult and a different color post insult. The wetness indicator may be appearing or disappearing graphics, such that a graphic object appears or disappears upon insult. Examples of suitable wetness indicators are disclosed in U.S. Pub. No. 2010/0262099.

Further, the leg cuffs 64 of the first and second chassis 38 may have identical component cross sectional order and disposition in at least one of the front waist region 26, back waist region 28, and crotch region 30. The distance from the left outer cuff distal edge to a right outer cuff distal edge may be the same. The distance from the left inner cuff proximal edge to left outer cuff distal edge may be the same. In some embodiments, the distance from the left inner cuff proximal edge to the right inner cuff proximal edge is the same. In some embodiments, the lengths of the inner and outer cuffs are the same.

In some embodiments, different size offerings in an array may have identical or substantially identical chassis as the flaps or belts may be used to enable the absorbent article to fit different sized wearers. For example, first and second absorbent articles may have identical chassis (compositionally, dimensionally, cross-sectionally), but the first article may have a different length due to disposition of the belts, such that the first article may be targeted to fit a smaller (in height, weight, or waist circumference) wearer than the second article. As a second example, first and second absorbent articles may have identical chassis (compositionally, dimensionally, cross-sectionally), but the first article may have a different length and/or width due to the size of the belts, such that the first article may be targeted to fit a smaller wearer than the second article.

In some embodiments, first and second absorbent articles may have identical chassis compositionally, but not dimensionally, and not cross-sectionally. In some embodiments, first and second absorbent articles may have identical chassis dimensionally, but not compositionally, and not cross-sectionally. In some embodiments, first and second absorbent articles may have identical chassis cross-sectionally, but not dimensionally, and not compositionally. In still other embodiments, first and second absorbent articles may have two, but not three of (1) compositionally, (2) dimensionally, and (3) cross-sectionally identical chassis.

### EXAMPLE ARRAYS

Further, absorbent article arrays of the present disclosure may be offered in Arrays 1-4, which are non-limiting examples, to accomplish the objects outlined in this application:

| ARRAY 1 (Inventive) | | | | | | | | | |
|---|---|---|---|---|---|---|---|---|---|
| | Front Belt width (mm) (124) | Back Belt width (mm) (126) | Front Belt Length (mm) (26) | Back Belt Length (mm) (28) | Product Rise (mm) (122) | Insert Length (mm) (128) | Insert Placement (mm) (130) | Number of Elastics in the Front Region | Number of Elastics in the Back Region |
| Article 1 | 648 | 648 | 179 | 193 | 658 | 471 | 70 | 27 | 33 |
| Article 2 | 728 | 728 | 196 | 213 | 752 | 471 | 117 | 29 | 39 |
| Article 3 | 808 | 808 | 219 | 238 | 860 | 471 | 171 | 32 | 46 |
| Article 4 | 648 | 648 | 200 | 214 | 700 | 548 | 52 | 30 | 36 |
| Article 5 | 728 | 728 | 211 | 227 | 780 | 548 | 92 | 31 | 41 |
| Article 6 | 808 | 808 | 219 | 238 | 860 | 548 | 132 | 32 | 46 |

| ARRAY 1 - Continued (Inventive) | | | | | | |
|---|---|---|---|---|---|---|
| | Belt length (26 or 28) to Belt width (124 or 126) Ratio | Belt Length (26 or 28) to Insert Placement (130) Ratio | Insert Placement (130) to Belt Width (124 or 126) Ratio | Product Rise (122) to Belt Length (26 or 28) Ratio | Insert Placement (130) to Product Rise (122) Ratio | Insert Length (128) to Product Rise (122) Ratio |
| Article 1 | 0.28 | 2.56 | 0.11 | 0.39 | 0.11 | 0.72 |
| Article 2 | 0.27 | 1.68 | 0.16 | 0.60 | 0.16 | 0.63 |
| Article 3 | 0.27 | 1.28 | 0.21 | 0.78 | 0.20 | 0.55 |
| Article 4 | 0.31 | 3.85 | 0.08 | 0.26 | 0.07 | 0.78 |
| Article 5 | 0.29 | 2.29 | 0.13 | 0.44 | 0.12 | 0.70 |
| Article 6 | 0.27 | 1.66 | 0.16 | 0.60 | 0.15 | 0.64 |

| ARRAY 2 (Inventive) | | | | | | | | | |
|---|---|---|---|---|---|---|---|---|---|
| | Front Belt width (mm) (124) | Back Belt width (mm) (126) | Front Belt Length (mm) (26) | Back Belt Length (mm) (28) | Product Rise (mm) (122) | Insert Length (mm) (128) | Insert Placement (mm) (130) | Number of Elastics in the Front Region | Number of Elastics in the Back Region |
| Article 1 | 645-655 | 645-655 | 175-185 | 190-200 | 650-660 | 465-475 | 65-75 | 25-32 | 30-35 |
| Article 2 | 725-735 | 725-735 | 190-200 | 210-220 | 750-760 | 465-475 | 110-120 | 27-32 | 37-42 |
| Article 3 | 805-815 | 805-815 | 215-225 | 235-245 | 855-865 | 465-475 | 165-175 | 30-35 | 44-49 |
| Article 4 | 645-655 | 645-655 | 195-205 | 210-220 | 695-705 | 545-555 | 45-55 | 28-33 | 34-39 |
| Article 5 | 725-735 | 725-735 | 205-215 | 225-235 | 775-785 | 545-555 | 85-95 | 29-34 | 38-42 |
| Article 6 | 805-815 | 805-815 | 215-225 | 235-245 | 855-865 | 545-555 | 125-135 | 29-34 | 43-48 |

| ARRAY 3 Exemplary (Comparative) | | | | | | | | | |
|---|---|---|---|---|---|---|---|---|---|
| | Front Belt width (mm) (124) | Back Belt width (mm) (126) | Front Belt Length (mm) (26) | Back Belt Length (mm) (28) | Product Rise (mm) (122) | Insert Length (mm) (128) | Insert Placement (mm) (130) | Number of Elastics in the Front Region | Number of Elastics in the Back Region |
| Article 1 | 650 | 650 | 158 | 172 | 616 | 470 | 44 | 22-26 | 28-32 |
| Article 2 | 730 | 730 | 175 | 192 | 710 | 470 | 96 | 24-28 | 34-38 |
| Article 3 | 650 | 650 | 138 | 152 | 576 | 430 | 44 | 18-22 | 24-28 |
| Article 4 | 730 | 730 | 155 | 172 | 670 | 430 | 96 | 22-24 | 32-36 |

| ARRAY 3 - Continued (Comparative) | | | | | | |
|---|---|---|---|---|---|---|
| | Belt length (26 or 28) to Belt width (124 or 126) Ratio | Belt Length (26 or 28) to Insert Placement (130) Ratio | Insert Placement (130) to Belt Width (124 or 126) Ratio | Product Rise (122) to Belt Length (26 or 28) Ratio | Insert Placement (130) to Product Rise (122) Ratio | Insert Length (128) to Product Rise (122) Ratio |
| Article 1 | 0.24 | 3.59 | 0.07 | 0.28 | 0.07 | 0.76 |
| Article 2 | 0.24 | 1.82 | 0.13 | 0.55 | 0.14 | 0.66 |
| Article 3 | 0.21 | 3.14 | 0.07 | 0.32 | 0.08 | 0.75 |
| Article 4 | 0.21 | 1.61 | 0.13 | 0.62 | 0.14 | 0.64 |

| ARRAY 4 (Inventive) | | | | | | | | | |
|---|---|---|---|---|---|---|---|---|---|
| | Front Belt width (mm) (124) | Back Belt width (mm) (126) | Front Belt Length (mm) (26) | Back Belt Length (mm) (28) | Product Rise (mm) (122) | Insert Length (mm) (128) | Insert Placement (mm) (130) | Number of Elastics in the Front Region | Number of Elastics in the Back Region |
| Article 1 | 650 | 650 | 180 | 195 | 655 | 470 | 70 | 25-32 | 30-35 |
| Article 2 | 730 | 730 | 195 | 215 | 755 | 470 | 115 | 27-32 | 37-42 |
| Article 3 | 810 | 810 | 220 | 240 | 860 | 470 | 170 | 30-35 | 44-49 |
| Article 4 | 650 | 650 | 200 | 215 | 700 | 550 | 50 | 28-33 | 34-39 |
| Article 5 | 730 | 730 | 210 | 230 | 780 | 550 | 90 | 29-34 | 38-42 |
| Article 6 | 810 | 810 | 220 | 240 | 860 | 550 | 130 | 29-34 | 43-48 |

| ARRAY 4 - Continued (Inventive) | | | | | | |
|---|---|---|---|---|---|---|
| | Belt length (26 or 28) to Belt width (124 or 126) Ratio | Belt Length (26 or 28) to Insert Placement (130) Ratio | Insert Placement (130) to Belt Width (124 or 126) Ratio | Product Rise (122) to Belt Length (26 or 28) Ratio | Insert Placement (130) to Product Rise (122) Ratio | Insert Length (128) to Product Rise (122) Ratio |
| Article 1 | 0.28 | 2.57 | 0.11 | 0.39 | 0.11 | 0.72 |
| Article 2 | 0.27 | 1.70 | 0.16 | 0.59 | 0.15 | 0.62 |
| Article 3 | 0.27 | 1.29 | 0.21 | 0.77 | 0.20 | 0.55 |
| Article 4 | 0.31 | 4.00 | 0.08 | 0.25 | 0.07 | 0.79 |
| Article 5 | 0.29 | 2.33 | 0.12 | 0.43 | 0.12 | 0.71 |
| Article 6 | 0.27 | 1.69 | 0.16 | 0.59 | 0.15 | 0.64 |

| ARRAY 5: Comparative (Non-Inventive, currently marketed) | | | | | | | |
|---|---|---|---|---|---|---|---|
| | Front Belt width (mm) (124) | Back Belt width (mm) (126) | Front Belt Length (mm) (26) | Back Belt Length (mm) (28) | Product Rise (mm) (122) | Insert Length (mm) (128) | Insert Placement (mm) (130) |
| Article 1 | 580 | 580 | 140 | 140 | 580 | 460 | 50 |
| Article 2 | 640 | 640 | 170 | 170 | 760 | 490 | 100 |

| ARRAY 5 - Continued: Comparative (Non-Inventive, currently marketed) | | | | | | |
|---|---|---|---|---|---|---|
| | Belt length (26 or 28) to Belt width (124 or 126) Ratio | Belt Length (26 or 28) to Insert Placement (130) Ratio | Insert Placement (130) to Belt Width (124 or 126) Ratio | Product Rise (122) to Belt Length (26 or 28) Ratio | Insert Placement (130) to Product Rise (122) Ratio | Insert Length (128) to Product Rise (122) Ratio |
| Article 1 | 0.24 | 2.80 | 0.09 | 0.36 | 0.09 | 0.79 |
| Article 2 | 0.27 | 1.70 | 0.16 | 0.59 | 0.13 | 0.64 |

| ARRAY 6: Comparative (Non-Inventive, currently marketed) | | | | | | | |
|---|---|---|---|---|---|---|---|
| | Front Belt width (mm) (124) | Back Belt width (mm) (126) | Front Belt Length (mm) (26) | Back Belt Length (mm) (28) | Product Rise (mm) (122) | Insert Length (mm) (128) | Insert Placement (mm) (130) |
| Article 1 | 700 | 700 | 190 | 190 | 750 | 410 | 120 |
| Article 2 | 760 | 760 | 240 | 240 | 850 | 410 | 170 |
| Article 3 | 800 | 800 | 200 | 200 | 900 | 410 | 140 |

| ARRAY 6 - Continued: Comparative (Non-Inventive, currently marketed) | | | | | | |
|---|---|---|---|---|---|---|
| | Belt length (26 or 28) to Belt width (124 or 126) Ratio | Belt Length (26 or 28) to Insert Placement (130) Ratio | Insert Placement (130) to Belt Width (124 or 126) Ratio | Product Rise (122) to Belt Length (26 or 28) Ratio | Insert Placement (130) to Product Rise (122) Ratio | Insert Length (128) to Product Rise (122) Ratio |
| Article 1 | 0.27 | 1.58 | 0.17 | 0.63 | 0.16 | 0.55 |
| Article 2 | 0.32 | 1.41 | 0.22 | 0.71 | 0.20 | 0.48 |
| Article 3 | 0.25 | 1.43 | 0.18 | 0.7 | 0.16 | 0.46 |

With regard to these arrays, it should be noted that the present disclosure reveals the importance of portion of the product rise (i.e., article length (122), also known as pitch) that is covered by the length of central chassis 38 (i.e., insert length (128)).

The dimensions and values disclosed herein are not to be understood as being strictly limited to the exact numeral values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A plurality of packages oriented in proximity to each other in a given area of a retail store, the plurality comprising:
a first package comprising a first disposable absorbent article, the first disposable absorbent article comprising a first topsheet (58), a first backsheet (60), a first absorbent core (62) and a first pair of side seams (32a, 32b), the first disposable absorbent article being a first size and in closed form;
a second package comprising a second disposable absorbent article, the second disposable absorbent article comprising a second topsheet, a second backsheet, a second absorbent core and a second pair of side seams, the second disposable absorbent article being a second size and in closed form;
wherein the second size is larger than the first size;
wherein the first and second disposable absorbent articles have a central chassis length (128) to product length (122) ratio from 0.55 to 0.64; and
wherein the first and second packages comprise the same brand name and/or sub-brand name.

2. The plurality of packages according to claim 1, further comprising a third package comprising a third disposable absorbent article, the third disposable absorbent article comprising a third topsheet, a third backsheet, a third absorbent core and a third pair of side seams, the third disposable absorbent article being a third size and in closed form, and wherein the first, second, and third packages comprise the same brand name and/or sub-brand name.

## Patentansprüche

1. Vielzahl von Verpackungen, die in einem bestimmten Bereich eines Einzelhandelsgeschäfts nahe beieinander ausgerichtet sind, die Vielzahl umfassend:
eine erste Verpackung, umfassend einen ersten Einweg-Absorptionsartikel, der erste Einweg-Absorptionsartikel umfassend eine erste Oberschicht (58), eine erste Unterschicht (60), einen ersten Absorptionskern (62) und ein erstes Paar von Seitennähten (32a, 280b), wobei der erste Einweg-Absorptionsartikel eine erste Größe vorweist und in einer geschlossenen Form vorliegt;
eine zweite Verpackung, umfassend einen zweiten Einweg-Absorptionsartikel, der zweite Einweg-Absorptionsartikel umfassend eine zweite Oberschicht, eine zweite Unterschicht, einen zweiten Absorptionskern und ein zweites Paar von Seitennähten, wobei der zweite Einweg-Absorptionsartikel eine zweite Größe vorweist und in einer geschlossenen Form vorliegt;
wobei die zweite Größe größer als die erste Größe ist;
wobei der erste und der zweite Einweg-Absorptionsartikel ein Verhältnis von zentraler Grundeinheitlänge (128) zu Produktlänge (122) von 0,55 bis 0,64 aufweisen; und
wobei die erste und die zweite Verpackung den gleichen Markennamen und/oder Untermarkennamen umfassen.

2. Vielzahl von Verpackungen nach Anspruch 1, ferner umfassend eine dritte Verpackung, umfassend einen dritten Einweg-Absorptionsartikel, der dritte Einweg-Absorptionsartikel umfassend eine dritte Oberschicht, eine dritte Unterschicht, einen dritten Absorptionskern und ein drittes Paar von Seitennähten, wobei der dritte Einweg-Absorptionsartikel eine dritte Größe vorweist und in einer geschlossenen Form vorliegt, und wobei die erste, die zweite und die dritte Verpackung den gleichen Markennamen und/oder Untermarkennamen umfassen.

## Revendications

1. Pluralité de paquets orientés à proximité les uns des autres dans une zone donnée d'un magasin de détail, la pluralité comprenant :
un premier conditionnement comprenant un premier article absorbant jetable, le premier article absorbant comprenant une première feuille de dessus (58), une première feuille de fond (60), une première âme absorbante (62) et une première paire de jointures latérales (32a, 32b), le premier article absorbant jetable étant une première taille et sous une forme fermée ;
un deuxième conditionnement comprenant un deuxième article absorbant jetable, le deuxième article absorbant comprenant une deuxième feuille de dessus, une deuxième feuille de fond, une deuxième âme absorbante et une deuxième paire de jointures latérales, le deuxième article absorbant étant une deuxième taille et de forme fermée ;
dans laquelle la deuxième taille est supérieure à la première taille ;
dans laquelle le premier et le deuxième articles absorbants jetables ont un rapport entre la longueur du châssis central (128) et la longueur du produit (122) compris entre 0,55 et 0,64 ; et
dans laquelle les premier et deuxième conditionnements comprennent le même nom de marque et/ou nom de sous-marque.

2. Pluralité de conditionnements selon la revendication 1, comprenant en outre un troisième conditionnement comprenant un troisième article absorbant jetable, le troisième article absorbant jetable comprenant une troisième feuille supérieure, une troisième feuille postérieure, une troisième âme absorbante et une troisième paire de coutures latérales, le troisième article absorbant jetable étant d'une troisième taille et sous forme fermée, et dans lequel le premier, le deuxième et le troisième conditionnement comprennent le même nom de marque et/ou nom de sous-marque.
